Europäisches Patentamt

European Patent Office

Office européen des brevets

(19)

(11) Veröffentlichungsnummer: **0 222 137
B1**

(12) **EUROPÄISCHE PATENTSCHRIFT**

(45) Veröffentlichungstag der Patentschrift:
19.09.90

(21) Anmeldenummer: 86113619.0

(22) Anmeldetag: 02.10.86

(51) Int. Cl.⁵: **A61M 25/00**, A61N 1/05

(54) Sonde für die Behandlung von Hohlorganen mit elektrischem Strom.

(30) Priorität: 04.10.85 AT 2881/85

(43) Veröffentlichungstag der Anmeldung:
20.05.87 Patentblatt 87/21

(45) Bekanntmachung des Hinweises auf die Patenterteilung:
19.09.90 Patentblatt 90/38

(84) Benannte Vertragsstaaten:
AT BE CH DE ES FR GB GR IT LI LU NL SE

(56) Entgegenhaltungen:
WO-A-82/00768
DE-A- 1 919 246
DE-A- 3 228 849
DE-A- 3 327 561
US-A- 3 070 132

(73) Patentinhaber: Nashef, Basem Dr., Geylinggasse 30,
A-1130 Wien(AT)
Patentinhaber: Urban, Gerald Dr., Rembrandtstrasse 19,
A-1020 Wien(AT)
Patentinhaber: Kovac, Werner Dr.,
Mariahilferstrasse 127, A-1060 Wien(AT)

(72) Erfinder: Nashef, Basem Dr., Geylinggasse 30,
A-1130 Wien(AT)
Erfinder: Urban, Gerald Dr., Rembrandtstrasse 19,
A-1020 Wien(AT)
Erfinder: Kovac, Werner Dr., Mariahilferstrasse 127,
A-1060 Wien(AT)

(74) Vertreter: Selting, Günther, Dipl.-Ing. et al,
Patentanwälte von Kreisler, Selting, Werner
Deichmannhaus am Hauptbahnhof, D-5000 Köln 1(DE)

## Beschreibung

Insbesondere in der Speiseröhre (Oesophagus) treten manchmal Varicen (Krampfadern) auf, welche zu Blutungen führen. Solche Blutungen sind oft schwer zu stillen und stellen daher eine akute Lebensgefahr dar. Die Erfindung bezieht sich auf eine Sonde für die Behandlung von eine Schleimhaut aufweisenden Hohlorganen, insbesondere der Speiseröhre, mit elektrischem Strom, insbesondere zur Behandlung von Varicen. Es sind beispielsweise für die Behandlung der Speiseröhre starre Sonden aus Silberblech bekannt. Diese Sonden werden in die Speiseröhre eingeführt und durch Einwirkung von elektrischem Strom wird eine Thrombosierung der Varicen verursacht. Durch diese verdickte Schleimhaut werden die Varicen geschützt und damit Blutungen verhindert. Zu diesem Zweck aber muß die Sonde an der Schleimhaut satt anliegen, da andernfalls eine Stromübertragung nicht möglich ist und außerdem Verletzungsgefahr besteht. Ein solches sattes Anliegen ist bei einer starren Sonde nicht gewährleistet.

Eine als WO-A-82/00768 bekannte Sonde, von der der Oberbegriff des Patentanspruchs 1 ausgeht, wird in ein Blutgefäß eingeführt. Die Sonde weist einen aufblasbaren Bereich auf, in dem mehrere Elektrodenfelder angeordnet sind. Wenn dieser Bereich aufgeblasen wird, legen sich die elektrisch leitenden Elektrodenfelder an die Wand des Gefäßes an. Die Elektrodenfelder sind an mehrere unterschiedliche Stromquellen angeschlossen, welche einen gemeinsamen Pol haben. Dieser gemeinsame Pol ist mit einer plattenförmigen Gegenelektrode verbunden, die auf die Haut des Patienten gelegt wird. Mit der Vorrichtung sollen oesophageale Varicen oder Hämorrhoiden in Blutgefäßen behandelt werden. Dabei werden Ströme erzeugt, die von den Elektrodenfeldern aus durch die Gefäßwand hindurchgehen.

Aus DE-A-33 27 561 ist eine Oesophaguselektrodensonde bekannt, die aus einem Schlauch besteht, welcher ringförmige Metallelektroden aufweist. Einige dieser Elektroden dienen zur EKG-Ableitung und eine Elektrode ist eine Stimulationselektrode. Die Sonde dient der temporären Elektrostimulation des Herzens, insbesondere in Notfallsituationen.

Es ist bisher keine Sonde bekannt, die einen gezielten Stromfluß entlang der Schleimhaut eines Hohlorganes bewirkt.

Die Erfindung stellt sich nun zur Aufgabe, eine Sonde zu schaffen, welche eine einwandfreie Behandlung der Innenflächen von Hohlorganen, und zwar insbesondere der Speiseröhre, ermöglicht. Mit dieser Ausbildung soll eine Entzündung der Schleimhaut der Speiseröhre hervorgerufen werden, welche, insbesondere bei mehrmaliger Behandlung, zu einer Verdickung der Schleimhaut führt.

Die Lösung dieser Aufgabe erfolgt erfindungsgemäß mit den Merkmalen des Patentanspruchs 1.

Dadurch, daß die Sonde aus flexiblem, dehnbarem Material, beispielsweise Gummi, besteht, wird ein Anliegen der Sonde an allen Stellen der Schleimhaut weitgehend begünstigt, und dadurch, daß die Oberfläche der Sonde eine flexible Schicht aus einem elektrisch leitenden, biologisch unbedenklichen Material aufweist, wird die Flexibilität und Dehnbarkeit der Sonde selbst nicht beeinträchtigt. Es wird somit die Zuführung des elektrischen Stromes an allen Stellen der Schleimhaut weitgehend begünstigt.

Wesentlich ist, daß die elektrisch leitende Schicht an der Schleimhaut möglichst satt anliegt, damit an allen Stellen der Schleimhaut die Wirkung des Stromes erreicht wird. Dies wird durch die flexible Ausbildung der Sonde und der Schicht begünstigt. Gemäß einer bevorzugten Ausführungsform der Erfindung ist weiters jedoch die Sonde im Bereich der elektrisch leitenden Beschichtung aufweitbar, so daß durch das Aufweiten der Sonde die elektrisch leitende Schicht an allen Stellen satt an die Schleimhaut angedrückt wird. Es ist hierbei wichtig, daß trotz der bei der Aufweitung der Sonde entstehenden Dehnung der elektrische Kontakt zwischen den metallischen Teilchen in der Matrix der Schicht aufrechterhalten bleibt. Dies wird z.B. dadurch erreicht, daß das Metall, (insbesondere Silber), in Form eines Netzwerkes aus feinsten Fäden in einer Polymermatrix eingebettet ist. Ein feines Netzwerk gewährleistet den elektrischen Kontakt der elektrisch leitenden Teilchen über die gesamte Oberfläche der Schicht.

Nach der Erfindung ist die elektrisch leitende Schicht in voneinander elektrisch isolierte Felder unterteilt, wobei zwei elektrische Leitungen in der Sonde geführt sind, welche an verschiedene Pole der Gleichstromquelle angeschlossen sind und von welchem je eine mit einem Feld oder einer Gruppe von Feldern elektrisch verbunden ist. Der Strom fließt somit über die Schleimhaut von einem Feld zum anderen oder von einer Gruppe von Feldern zur anderen Gruppe.

Gemäß der Erfindung kann eine elektrisch leitende Schicht als metallische Schicht auf die Sonde aufgedampft oder in Form einer Metall in fein verteilter Form enthaltenden Matrix aufgestrichen sein, so daß im Falle des Aufdampfens eine dünne und im Falle der Einbettung der metallischen Teilchen in eine Matrix eine flexible Schicht erhalten wird, welche die Flexibilität der Sonde nicht beeinträchtigt. Gemäß der Erfindung besteht die elektrisch leitende Schicht vorzugsweise aus Silber, welches sich als biologisch unbedenkliches Material bewährt hat. Gemäß einer bevorzugten Ausführungsform der Erfindung ist auf der Silberschicht eine Silberchloridschicht ausgebildet. Diese Silberchloridschicht, die beispielsweise auf elektrolytischem oder chemischem Weg aus dem Silber gebildet werden kann, hat den Effekt, daß Polarisationserscheinungen vermieden werden und daß der elektrische Übergangswiderstand von der Silberschicht auf die Schleimhaut, unabhängig von der Stromrichtung, verringert wird. Vorzugsweise wird Gleichstrom verwendet und in diesem Fall hat die Silberchloridschicht eine besonders vorteilhafte Wirkung.

Gemäß der Erfindung ist an die in der Sonde geführte elektrische Leitung wenigstens ein in die elektrisch leitende Schicht eingebetteter oder an der Oberfläche derselben liegender Verteilerdraht aus einem biologisch unbedenklichen Metall, insbesondere Silber, angeschlossen. Die Anordnung des Verteilerdrahtes bringt den Vorteil mit sich, daß durch diesen Verteilerdraht über die gesamte Länge der elektrisch leitenden Schicht der Strom dieser Schicht zugeführt wird, da durch diese Anordnung der Kontaktwiderstand Silberdraht zur Silberschicht verringert wird. Gemäß der Erfindung kann der Verteilerdraht in Windungen um die Sonde gelegt sein.

Gemäß einer weiteren bevorzugten Ausführungsform der Erfindung weist die Sonde an ihrem freien Ende einen aufweitbaren Ballon auf. Wenn die Sonde in die Speiseröhre eingeführt wird, gelangt der Ballon in den Magen. Nach Aufweitung des Ballons kann dann die Sonde so weit zurückgezogen werden, daß der aufgeweitete Ballon an die Eintrittsstelle der Speiseröhre in den Magen anstößt. Es ist dadurch der richtige Sitz der Sonde kontrollierbar und es wird weiters vermieden, daß die Sonde im Zuge der Behandlung aus der Speiseröhre herausgezogen wird. Es kommen nun auch Varicen im Bereich des Eintritts der Speiseröhre in den Magen (Cardia) vor. Gemäß der Erfindung kann auch der Ballon an seiner der Sonde zugewendeten Seite eine Silber- oder dergleichen -Schicht aufweisen, welche über wenigstens einen Verteilerdraht mit Strom versorgbar ist. Es wird dadurch auch die Behandlung solcher im Bereich des Eintrittes der Speiseröhre in den Magen (Cardia) auftretender Varicen in gleicher Weise ermöglicht. Vorzugsweise weist gemäß der Erfindung die Sonde an ihrem Handhabungsende einen Druckflüssigkeitsanschluß für die Aufweitung des aufweitbaren Sondenabschnittes und des Ballons auf. Als Druckflüssigkeit wird im allgemeinen Wasser verwendet.

Es kann die elektrisch leitende Schicht in voneinander elektrisch isolierte Felder unterteilt sein und jedem Feld eine gesonderte, in der Sonde geführte elektrische Leitung zugeordnet sein.

In an sich bekannter Weise kann auch eine für die Behandlung der Speiseröhre bestimmte Sonde einen den Ballon durchsetzenden, in den Magen mündenden Schlauch aufweisen, durch den dem Magen Flüssigkeiten zugeführt oder abgesaugt werden können.

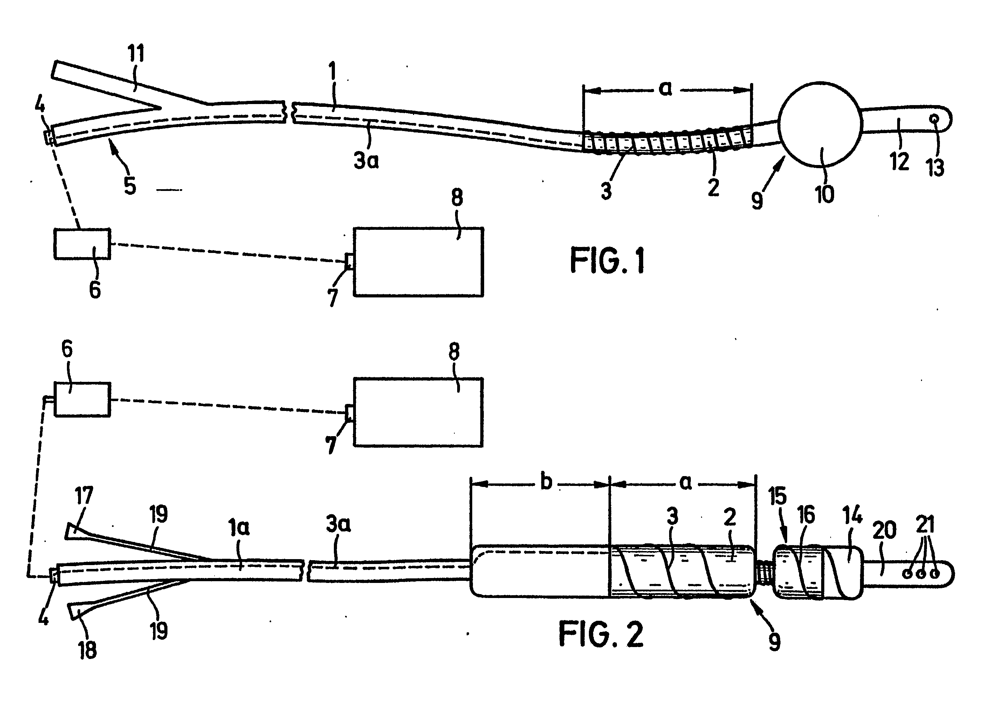

In der Zeichnung ist die Erfindung anhand von dem Ausführungsbeispiel nach Fig. 2 schematisch erläutert.

Fig. 1 zeigt eine Sonde um das Verständnis der Erfindung zu erleichtern.

Fig. 2 zeigt eine Ausbildung einer aufweitbaren Sonde gemäß der Erfindung.

Die Sonde nach Fig. 1 besteht aus einem flexiblen Schlauch 1, beispielsweise aus Gummi. In einem Bereich a weist die Sonde eine schraffiert angedeutete Schicht 2 aus Silber bzw. in einer Matrix eingeschlossene Silberteilchen auf. Ein Verteilerdraht 3 ist in Windungen um die Schicht 2 geführt und steht mit dieser in elektrisch leitender Verbindung. Dieser Verteilerdraht 3 kann auch in die Matrix der Schicht 2 eingebettet sein, so daß er nicht unmittelbar mit der Schleimhaut in Berührung gelangt. Innerhalb der Sonde 1 ist ein elektrischer Leitungsdraht 3a angeordnet, welcher den Verteilerdraht 3 mit einer Steckbuchse 4 am Handhabungsende 5 der Sonde verbindet. Die Steckbuchse 4 ist an eine Stromquelle 6 angeschlossen, deren anderer Pol mit einer Steckbuchse 7 einer Hautelektrode 8 verbunden ist. Diese Hautelektrode 8 kann beispielsweise auf die Brust aufgelegt werden. An ihrem freien Ende 9 weist die Sonde 1 einen aufweitbaren Ballon 10 auf, welcher über einen nicht dargestellten Druckwasseranschluß durch Druckwasser aufgeweitet werden kann. Die Sonde wird, wenn sie zur Behandlung der Speiseröhre dient, durch die Speiseröhre bis in den Magen eingeführt, so daß der aufgeweitete Ballon 10 ein Herausgleiten der Sonde verhindert. Beim Herausziehen der Sonde wird der Ballon 10 drucklos gemacht.

Es ist noch am Handhabungsende 5 der Sonde ein weiterer Rohr- oder Schlauchanschluß 11 vorgesehen. Der Sondenschlauch oder ein in der Sonde geführter gesonderter Schlauch 12 ist durch den Ballon 10 hindurchgeführt. Über den Anschluß 11 können nun Flüssigkeiten in den Magen eingeführt werden, welche durch eine Öffnung 13 in den Magen austreten können, oder es kann Flüssigkeit abgesaugt werden.

Eine solche Sonde kann beispielsweise zur Behandlung der Speiseröhre von Kindern, jedoch auch zur Behandlung der Speiseröhre von Erwachsenen dienen.

In Fig. 2 ist eine Ausführungsform der Sonde gemäß der Erfindung dargestellt. Der Bereich a, welcher die Silberbeschichtung 2 trägt, ist aufweitbar ausgebildet und kann durch Druckwasser aufgeweitet werden. Es ist auch hier ein Verteilerdraht 3 vorgesehen, welcher in die Matrix der Silberschicht 2 eingebettet ist. Der Verteilerdraht 3 ist derart in Windungen gelegt, daß er der Aufweitung des Bereiches a folgen kann, ohne zu brechen. Der Verteilerdraht 3 ist über einen nicht dargestellten, in der Sonde 1a geführten Leitungsdraht mit einer Steckbuchse 4 verbunden, welche mit der Stromquelle 6 in Verbindung steht. Der mit der Silberschicht versehene Bereich a muß nur so lang sein, soweit sich der zu behandelnde Bereich der Schleimhaut erstreckt. Es kann aber auch ein anschließender Bereich b, welcher keine Beschichtung aufweist, in gleicher Weise durch Wasserdruck aufgeweitet werden, um einen sicheren Sitz der Sonde in der Speiseröhre zu gewährleisten.

Am freien Ende 9 der Sonde 1a ist wieder ein aufweitbarer Ballon 14 vorgesehen. Bei der Ausführungsform nach Fig. 2 weist dieser Ballon 14 an der der Sonde 1a zugewendeten Seite 15 eine Silberbeschichtung auf, welcher der Strom wieder über einen Verteilerdraht 16 zu geführt wird. Der beschichtete Bereich 15 des Ballons legt sich an die Magenwandung im Bereich der Mündung der Speiseröhre (Cardia) an, und es ist auf diese Weise möglich, auch Varicen in diesem Bereich des Magens zu behandeln. Es sind auch hier Anschlüsse 17 und 18 vorgesehen, über welche Flüssigkeit für den Magen durch die Sonde 1a oder durch in der Sonde 1a geführte Schläuche 19 zu einem den Ballon 14 durchsetzenden Schlauch 20 geführt werden kann. 21 sind Löcher für den Austritt der Flüssigkeit.

Die in Fig. 1 und 2 dargestellte Sonde ist für die Behandlung der Speiseröhre bestimmt. Die erfindungsgemäße Sonde kann aber auch für andere Hohlorgane verwendet werden, wie z.B. für die Behandlung von Hämorrhoiden.

## Patentansprüche

1. Sonde für die Behandlung der Schleimhaut von Hohlorganen, insbesondere der Speiseröhre, mit elektrischem Strom, insbesondere zur Behandlung von Varicen, wobei die Sonde schlauchförmig ausgebildet ist, aus flexiblem, vorzugsweise dehnbarem Material, insbesondere Gummi, besteht, und wenigstens auf einem aufweitbaren Bereich ihrer Oberfläche eine elektrisch leitende flexible Schicht (2) aus einem biologisch unbedenklichen Material aufweist, die in mehrere voneinander elektrisch isolierte Felder unterteilt ist und über mindestens eine isoliert geführte elektrische Leitung (3a) an eine elektrische Stromquelle (6) angeschlossen ist, dadurch gekennzeichnet, daß in der Sonde zwei elektrische Leitungen (3, 16) geführt sind, die an verschiedene Pole der Stromquelle (6) angeschlossen sind und von denen je eine mit einem Feld oder einer Gruppe von Feldern verbunden ist.

2. Sonde nach Anspruch 1, dadurch gekennzeichnet, daß die elektrisch leitende Schicht (2) als metallische Schicht auf die Sonde aufgedampft oder in Form einer Metall in fein verteilter Form enthaltenden Matrix aufgestrichen oder in das Sondenmaterial eingearbeitet wird.

3. Sonde nach Anspruch 1 oder 2, dadurch gekennzeichnet, daß die elektrisch leitende Schicht (2) aus Silber besteht.

4. Sonde nach Anspruch 3, dadurch gekennzeichnet, daß auf der Silberschicht eine Silberchloridschicht ausgebildet ist.

5. Sonde nach einem der Ansprüche 1 bis 4, dadurch gekennzeichnet, daß an die in der Sonde geführte elektrische Leitung (3a) wenigstens ein in die elektrisch leitende Schicht (2) eingebetteter oder an der Oberfläche derselben liegender Verteilerdraht (3) aus einem biologisch unbedenklichen Metall, insbesondere Silber, angeschlossen ist.

6. Sonde nach Anspruch 5, dadurch gekennzeichnet, daß der Verteilerdraht (3) in Windungen um die Sonde gelegt ist.

7. Sonde nach einem der Ansprüche 1 bis 6, dadurch gekennzeichnet, daß das Silber oder dergleichen in Form eines Netzwerkes aus feinsten Fäden in einer Polymermatrix eingebettet ist.

8. Sonde nach einem der Ansprüche 1 bis 7, dadurch gekennzeichnet, daß sie in der Nähe ihres patientenseitigen vorderen Endes (9) einen aufweitbaren Ballon (10; 14) aufweist, welcher an seinem der Sonde zugewendeten rückseitigen Bereich (15) eine Silber- oder dergleichen -Schicht aufweist, welche über wenigstens einen Verteilerdraht mit Strom versorgbar ist.

9. Sonde nach einem der Ansprüche 1 bis 8, dadurch gekennzeichnet, daß sie an ihrem Handhabungsende einen Druckflüssigkeitsanschluß (18) für die Aufweitung des aufweitbaren Sondenabschnittes und des Ballons aufweist.

10. Sonde nach einem der Ansprüche 1 bis 9, dadurch gekennzeichnet, daß die elektrisch leitende Schicht in voneinander elektrisch isolierte Felder unterteilt ist und jedem Feld eine gesonderte, in der Sonde geführte elektrische Leitung zugeordnet ist.

## Claims

1. A probe for the treatment of the mucous membrane of hollow organs, particularly of the esophagus, with electric current, particularly for the treatment of varicose veins, the probe preferably being hose-shaped, consisting of flexible, preferably expansible material, particularly rubber, and having, at least on a part of its surface, an electrically conductive flexible coating (2) of a biologically safe material, which coating is subdivided into a plurality of fields, electrically insulated from each other, and is connected to an electric current source (6) via at least one insulated electric line (3a), characterized in that two electric wires (3, 16) are guided within said probe that are connected to different terminals of said current source (6) and each of which is respectively connected with one field or with a group of fields.

2. The probe according to claim 1, characterized in that the electrically conductive coating (2) is deposited by evaporation onto the probe as a metallic coating or is spread on the probe as a matrix containing finely distributed metal or is worked into the probe material.

3. The probe according to claim 1, characterized in that the electrically conductive coating (2) consists of silver.

4. The probe according to claim 3, characterized in that a layer of silver chloride is provided on the silver coating.

5. The probe according to one of claims 1 to 4, characterized in that at least one connection wire (3) embedded in the conductive coating (2) or positioned at the surface of the latter, which connection wire is made of a biologically safe material, particularly of silver, is connected to the electric line (3a) guided within the probe.

6. The probe according to claim 5, characterized in that the connection wire (3) is arranged in coils around the probe.

7. The probe according to one of claims 1 to 6, characterized in that the silver or the like is embedded in a polymer matrix as a netting of finest wires.

8. The probe according to one of claims 1 to 7, characterized in that it comprises an expansible balloon (10, 14) near its patient side front end (9), which balloon (14), at its rear section (15) facing the probe, comprises a coating of silver or the like to which current can be supplied via at least one connection wire (16).

9. The probe according to one of claims 1 to 8, characterized in that, at its operating end, it comprises a connection (18) for pressurized fluid for the expansion of the expansible section of the probe and of the balloon.

10. The probe according to one of claims 1 to 9, characterized in that the conductive coating is subdivided into electric fields electrically insulated from each other and a separate electric line guided within the probe is assigned to each field.

## Revendications

1. Sonde pour le traitement de la muqueuse des organes creux, notamment du tube digestif, à l'aide d'un courant électrique, notamment pour le traitement des varices, réalisée sous la forme d'un tuyau souple, en une matière flexible, de préférence extensible, notamment en caoutchouc, et comprenant, sur au moins une zone de sa surface qui est dilatable, une couche souple électriquement conductrice (2), en une matière inoffensive sur le plan biologique, qui est divisée en plusieurs zones isolées électriquement l'une de l'autre et est raccordée à une source électrique de courant (6) par au moins un fil électrique (3a) qui est guidé d'une manière isolée, caractérisée en ce que deux fils électriques (3, 16) sont guidés dans cette sonde, ces fils électriques étant raccordés à des pôles différents de la source de courant (6) et chacun d'eux étant relié à une zone ou un groupe de zones.

2. Sonde selon la revendication 1, caractérisée en ce que la couche électriquement conductrice (2) est déposée sur la sonde, sous la forme d'une couche métallique, par vaporisation ou par enduction sous la forme d'une matrice contenant un métal sous une forme finement divisée, ou bien encore est incorporée dans la matière de la sonde.

3. Sonde selon la revendication 1 ou 2, caractérisée en ce que la couche électriquement conductrice (2) est en argent.

4. Sonde selon la revendication 3, caractérisée en ce qu'une couche de chlorure d'argent est réalisée sur la couche d'argent.

5. Sonde selon l'une des revendications 1 à 4, caractérisée en ce qu'au fil électrique (3a) guidé dans la sonde, il est raccordé au moins un fil de distribution (3), en un métal inoffensif sur le plan biologique, notamment en argent, qui est enrobé dans la couche électriquement conductrice (2) ou se trouve sur la surface de celle-ci.

6. Sonde selon la revendication 5, caractérisée en ce que le fil de distribution (3) est posé en spires autour de la sonde.

7. Sonde selon l'une des revendications 1 à 6, caractérisée en ce que l'argent, ou analogue, est enrobé dans une matrice de polymère sous la forme d'un réseau de filaments extrêmement fins.

8. Sonde selon l'une des revendications 1 à 7, caractérisée en ce que, à proximité de son extrémité avant (9) dirigée vers le patient, elle comprend un ballon dilatable (10; 14) qui, sur sa zone arrière (15) tournée vers la sonde, comporte une couche d'argent, ou analogue, qui peut être alimentée en courant par au moins un fil de distribution.

9. Sonde selon l'une des revendications 1 à 8, caractérisée en ce que, à son extrémité de manipulation, elle comprend un raccord pour fluide sous pression (18) servant à la dilatation du tronçon dilatable de sonde et du ballon.

10. Sonde selon l'une des revendications 1 à 9, caractérisée en ce que la couche électriquement conductrice est divisée en zones électriquement isolées entre elles et en ce qu'il est associé à chaque zone un fil électrique particulier guidé dans la sonde.

FIG.1

FIG.2